# EUROPÄISCHE PATENTSCHRIFT

(11) **EP 0 694 258 B1**
(45) Veröffentlichungstag und Bekanntmachung des Hinweises auf die Patenterteilung: **05.09.2001**
(21) Anmeldenummer: 95109942.3
(22) Anmeldetag: 26.06.1995
(51) Int. Cl.: A01N 43/40, A01N 43/80

(54) **Konservierungsmittelmischung für diagnostische Testflüssigkeiten**
Mixture of preservatives for diagnostic test fluids
Mélange de préservatifs pour liquides de test diagnostiques

(30) Priorität: 27.06.1994 DE 4422374
(43) Veröffentlichungstag der Anmeldung: 31.01.1996
(73) Patentinhaber: Roche Diagnostics GmbH, 68305 Mannheim (DE)
(72) Erfinder: Kurrle-Weittenhiller, Angelika, Dr., D-82327 Tutzing (DE); Schmidt, Axel, Dr., D-80634 München (DE)
(74) Vertreter: Böhm, Brigitte, Dipl.-Chem. Dr.

(56) Entgegenhaltungen:
- EP-A- 0 443 821
- EP-A- 0 467 337
- EP-A- 0 608 913
- GB-A- 2 077 916
- GB-A- 2 230 190
- US-A- 5 283 005

## Beschreibung

Die vorliegende Erfindung betrifft Konservierungsmittelmischungen, die Verwendung bestimmter Konservierungsmittel oder Konservierungsmittelmischungen in Testflüssigkeiten diagnostischer Testkits sowie diagnostische Testkits, bei denen eine oder mehrere Testflüssigkeiten mit einem erfindungs emäßen Konservierungsmittel stabilisiert sind.

Geeignete Konservierungsmittel für diagnostische Testkits sind nicht leicht zu finden, da oft eine Beeinträchtigung der Bestimmung der nachzuweisenden oder zu quantifizierenden Substanz durch das Konservierungsmittel auftritt oder zu befürchten ist. Ein weiteres Problem liegt in der geringen Löslichkeit der Konservierungsmittel, welche auch nicht für alle der bisher bekannten Konservierungsmittel ausreichend ist, um einen Einsatz in diagnostischen Testkits zu erlauben. Schließlich haben sich viele der bisher bekannten Konservierungsmittelsubstanzen als ungeeignet erwiesen, da eine Eintrübung des Reagenz, eine gestörte Wiederfindung in Proben, Hämoglobinstörung bei blutenthaltenden Proben, ungenügende mikrobiologische Wirksamkeit, Instabilität des Reagenz bei Belastung und weitere Nachteile beobachtet wurden. Aus diesem Grunde wurde in diagnostischen Tests bevorzugt Natriumazid verwendet, welches bisher zumindest die geringsten Störungen der Tests, insbesondere von α-Amylasetests, zeigt .

Die Verwendung von Natriumazid bietet jedoch immer mehr Probleme. Ganz generell wird heute versucht, die Verwendung von derart giftigen Substanzen zu vermeiden, insbesondere da es sich bei Natriumazid um einen mutagenen, potentiell karzinogenen und cytotoxischen Stoff handelt, der darüber hinaus noch explosive Schwermetallazide bei der Lagerung bilden kann. Im übrigen ist die konservierende Wirkung von Natriumazid manchmal problematisch, da Resistenzen zunehmen und das Wirkungsspektrum nicht sehr breit ist. Insbesondere bei Durchführung eines Amylasetests tritt darüber hinaus eine Hämoglobinstörung auf, welche durch Komplexbildung von Hämoglobin mit Azid hervorgerufen wird. Diese Hämoglobinstörung konnte nur durch den Zusatz einer Entstörsubstanz teilweise behoben werden, was jedoch ebenfalls einen Nachteil darstellt, da wiederum eine weitere Substanz zugeführt wird, die selbst u.a. Störungen hervorrufen kann.

Aufgabe der vorliegenden Erfindung war es daher, eine Konservierung bereitzustellen, welche in diagnostischen Tests eingesetzt werden kann unter größtmöglicher Vermeidung von Störungen bei der Testdurchführung.

Erfindungsgemäß wird dies erreicht durch die Verwendung von Mercaptopyridin-N-oxid-Natriumsalz zusammen mit einer Verbindung aus der Gruppe der Isothiazolone zur Konservierung von Testflüssigkeiten diagnostischer Testkits und insbesondere von Testflüssigkeiten eines Amylasetests, sowie eine Konservierungsmittelmischung, insbesondere für die Konservierung von diagnostischen Testflüssigkeiten, welche das Natriumsalz von Mercaptopyridin-N-oxid zusammen mit einem 2-Alkyl-3-(2H)isothiazolon-hydrochlorid, dessen 2-Alkyl-Gruppierung ein bis acht Kohlenstoffatome umfaßt, in den anspruchgemäß angegebenen Mengen enthält.

Überraschenderweise hat man festgestellt, daß das Natriumsalz von Mercaptopyridin-N-oxid die diagnostische Bestimmung bestimmter Substanzen in Testflüssigkeiten, und insbesondere von α-Amylase in Körperflüssigkeiten nicht oder nur in vernachlässigbarer Weise beeinträchtigt. Mercaptopyridin-N-oxid-Natriumsalz stellt außerdem eine Verbindung dar, die für höhere Organismen nur wenig giftig ist und keinerlei Probleme bei der Handhabung verursacht.

Es wurden bereits microbizide Zusammensetzungen offenbart, die Isothiazolonderivate (EP-A2 0 431 752, EP-A1 0 608 913) oder 2-Pyridinthiol-1-oxid (Pyrithion) (GB 2 077 916, US Patent Nr. 5,283,005) enthalten. Eine erfindungsgemäße Kombination zur Konservierung diagnostischer Testflüssigkeiten wird dort jedoch nicht nahegelegt.

Eine britische Patentanmeldung, GB 2 230 190, lehrt Zusammensetzungen, die ein 2-Mercaptopyridin-1-oxidderivat und ein Isothiazolin(thi)onderivat enthalten. Es ist jedoch weder eine erfindungsgemäße Konservierungsmittelmischung noch eine Verwendung von Mercaptopyridin-N-oxid-Natriumsalz zusammen mit einen Isothiazolon zur Konservierung von Testflüssigkeiten diagnostischer Testkits offenbart oder nahegelegt.

Die europäische Anmeldung EP 0 443 821 offenbart die Stabilisierung von 3-Isothiazolonen unter anderem mit 2-Mercaptopyridin-N-oxid. Ein Hinweis auf eine Eignung zur Konservierung von diagnostischen Testflüssigkeiten oder auf bestimmte vorteilhafte mengenmäßige Beschränkungen bei der Kombination der Substanzen findet sich nicht.

Die EP-A2 0 467 337 schlägt eine Kombination von Konservierungsmitteln zum Konservieren von diagnostischen Tests vor, wobei die dort genannten Substanzen nicht die Kombination eines Mercaptopyridin-N-oxid-Natriumsalzes mit einem Isothiazolon umfassen.

Bei der Bestimmung von α-Amylase unter Verwendung der erfindungsgemäßen Konservierun smittelmischung hat sich herausgestellt, daß die Qualität der im Test verwendeten α-Glucosidase eine Rolle spielt. Als besonders geeignet hat sich eine α-Glucosidase aus einem thermostabilen Mikroorganismus erwiesen.

Durch Einsatz von zwei Konservierungsmitteln in der erfindungsgemäßen Konservierungsmittelmischung, also dem Mercaptopyridin-N-oxid und einer der weiteren genannten konservierenden Substanzen, bietet sich der weitere Vorteil, daß Resistenzentwicklungen verhindert werden und daß man im allgemeinen auch mit geringeren Mengen der jeweiligen Substanzen auskommen kann.

Als Verbindungen aus der Gruppe der Isothiazolone sind insbesondere 2-Alkyl-3- (2H) -isothiazolon-hydrochloride bevorzugt, die in der 2-Alkyl-Gruppierung 1 bis 8 Kohlenstoffatome enthalten. Eine besonders bevorzugte Verbindung ist das 2-Methyl-3-(2H)-isothiazolon-hydrochlorid (MIT) oder aber auch das 2-Methyl-4,5-trimethylen-4-isothiazolin-on.

Insbesondere mit einer dieser bevorzugten Konservierungsmittelmischung wird im Fall eines diagnostischen Amylasetests eine sehr viel geringere Hämoglobinstörung beobachtet als bei Verwendung von Natriumazid.

Die einzelnen Substanzen der Konservierungsmittelmischung sind in solchen Mengen vorhanden, daß sie in der zu konservierenden Testflüssigkeit vorzugsweise in folgenden Konzentrationen vorliegen:
Mercaptopyridin-N-oxid-Natriumsalz:
0,005 bis 0,1 % und besonders bevorzugt 0,008 bis 0,012 %;
Isothiazolon-Verbindung 0,005 bis 0,1 % und besonders bevorzugt 0,015 bis 0,025 %.

Die erfindungsgemäße Konservierungsmittelmischung weist also Vorteile dahingehend auf, daß sie nur geringste Störungen bei diagnostischen Tests verursacht und daher ohne weitere Entstörungsmaßnahmen eingesetzt werden kann, um die Flüssigkeiten in diagnostischen Testkits zu konservieren. S.e enthält darüber hinaus keine aufgrund ihrer Toxizität problematischen Verbindungen und durch die Kombination mindestens zweier konservierender Stoffe werden Resistenzbildungen verhindert und man kommt auch mit verhältnismäßig geringen Mengen der einzelnen Substanzen aus.

Weitere Gegenstände der vorliegenden Erfindung sind diagnostische Testkits, die als Konservierungsmittel einer oder mehrerer Testflüssigkeiten des Testkits Mercaptopyridin-N-oxid-Natriumsalz zusammen mit einer Verbindung der Gruppe der Isothiazolone enthalten. Besonders bevorzugt sind hierbei 2-Methyl-4,5-trimethylen-4-isothiazolin-on bzw. 2-Alkyl-3-(2H)-isothiazolon-hydrochloride mit 1 bis 8 C-Atomen in der 2-Alkylgruppe und insbesondere 2-Methyl-3-(2H)-isothiazolon-hydrochlorid (MIT).

Besonders bevorzugt handelt es sich bei dem erfindungsgemäßen diagnostischen Testkit, der mit Hilfe der erfindungsgemäßen Konservierungsmittelmischung bzw. der genannten Einzelverbindungen konserviert wird, um einen Testkit zur Bestimmung der α-Amylase. Andere Testkits unter Einsatz von Testflüssigkeiten, welche bereits in der Testpackung in flüssiger Form vorliegen und über längere Zeit stabilisiert werden müssen, sind jedoch auch im Rahmen der Erfindung geeignet. Die erfindungsgemäße Konservierungsmittelmischung findet in allen derartigen diagnostischen Testkits Anwendung und führt zu den bereits oben genannten Vorteilen gegenüber bisher bekannten Konservierungsmitteln oder Methoden zur Konservierung von Flüssigkeiten.

Die vorliegende Erfindung soll durch die folgenden Beispiele weiter erläutert werden.

### Beispiel 1 [Vergleichssubstanzen in eckigen Klammern]

Lösungen für die diagnostische Bestimmung von α-Amylase

| | | |
|---|---|---|
| Reagenz 1 | α-Glucosidase multifunktionell: (Fa. Toyobo, Japan) | 8 U/ml |
| | HEPES-Puffer | 105 mmol/l pH 7,1 |
| | NaCl | 52 mmol/l |
| | MgCl₂ | 10,5 mmol/l |
| | MIT [oder Bromnitrodioxan] | 0,02 % |
| | Mercaptopyridin-N-oxid, Na-Salz | 0,01 % |

| | | |
|---|---|---|
| Reagenz 2 | 4,6-Ethyliden-G7-pNP | 20 mmol/l |
| | HEPES-Puffer | 105 mmol/l pH 7,1 |
| | NaCl | 52 mmol/l |
| | MgCl₂ | 10,5 mmol/l |
| | MIT [oder Bromnitrodioxan:] | 0,02 % |
| | Mercaptopyridin-N-oxid, Na-Salz | 0,01 % |

### Beispiel 2

Durchführung der Bestimmung der Menge von α-Amylase (total und Pankreas-α-Amylase)

### Testprinzip

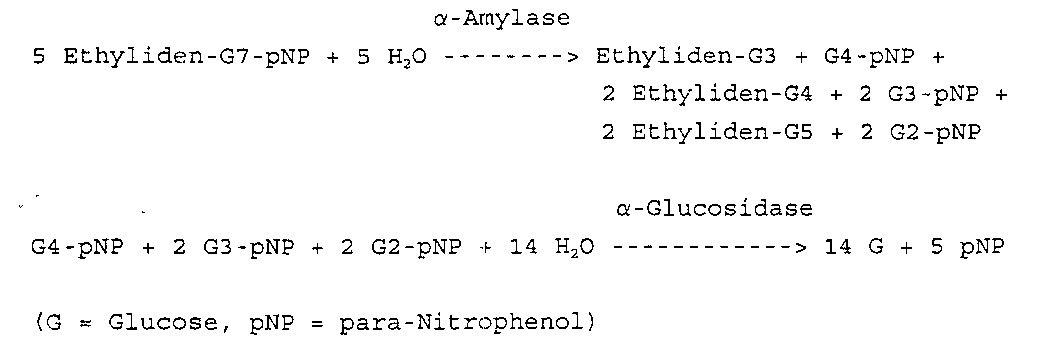

### Pankreasamylase:

Durch spezifische Antikörper wird die Speichel-α-Amylase gehemmt, so daß nur die Pankreas-α-Amylase an der oben beschriebenen Reaktion teilnimmt.
- Kalibration:: Std. 1 = 0,9 % NaCl, Std. 2 = Cfas
- Qualitätskontrolle:: PNU, PPU, PNE, PPE
- Probenmaterial:: Serum, EDTA-Plasma, Heparin-Plasma, Urin
- Verdünnungsgrenze :: 2000 U/l
- Reagenzien:: **T-α-Amylase:** Lösung 1: Enzymlösung, gebrauchsfertig
Lösung 2: Substratlösung, gebrauchsfertig
**P-α-Amylase:** Lösung 1: Enzym-Antikörper-Lösung gebrauchsfertig
Lösung 2: Substratlösung, gebrauchsfertig
- Bestimmungsansatz :: Wellenlänge: Hg 405 nm
Küvette: 1 cm Schichtdicke
Meßtemperatur: 37°C
Messung gegen Luft (Extinktionszunahme)

In eine Küvette pipettieren

| | Std. 1 | Std. 2 | Probe | Qk |
|---|---|---|---|---|
| Lösung 1 | 2,5 ml | 2,5 ml | 2,5 ml | 2,5 ml |

| Meßtemperatur überprüfen | | | | |
|---|---|---|---|---|
| NaCl | 0,1 ml | - | - | - |
| CFas | - | 0,1 ml | - | - |
| Serum/Plasmid/Urin | - | - | 0,1 ml | - |
| PNU/PPU/PNE/PPE/ | - | - | - | 0,1 ml |

| Mischen, 5 min bei Meßtemperatur inkubieren | | | | |
|---|---|---|---|---|
| Lösung 2 | 0,5 ml | 0,5 ml | 0,5 ml | 0,5 ml |
| Mischen, bei Meßtemperatur inkubierten E1 nach genau 3 min ablesen, E2 nach genau 6 min ablesen dE = E2 - E1 | | | | |

## Patentansprüche

1. VerwendungvonMercaptopyridin-N-oxid-Natriumsalzzusammen mit einer Verbindung aus der Gruppe der Isothiazolone zur Konservierung von Testflüssigkeiten diagnostischer Testkits und insbesondere von Testflüssigkeiten eines Amylasetests.

2. Verwendung nach Anspruch 1,
**dadurch gekennzeichnet,**daß
als Isothiazolon ein 2-Alkyl-3-(2H)-isothiazolon-hydrochlorid, dessen 2-Alkylgruppe ein bis acht C-Atome umfaßt, oder 2-Methyl-4,5-trimethylen-4-isothiazolinon eingesetzt wird.

3. Verwendung nach Anspruch 2,
**dadurch gekennzeichnet,**daß
2-Methyl-3-(2H)-isothiazolon-hydrochlorid eingesetzt wird.

4. Diagnostischer Testkit,
**dadurch gekennzeichnet,** daß
er als Konservierungsmittel einer oder mehrerer Testflüssigkeiten des Testkits Mercaptopyridin-N-oxid-Natriumsalz zusammen mit einer Verbindung aus der Gruppe der Isothiazolone enthält.

5. Diagnostischer Testkit nach Anspruch 4,
**dadurch gekennzeichnet,** daß
er als Verbindung aus der Gruppe der Isothiazolone ein 2-Alkyl-3-(2H)-isothiazolon-hydrochlorid, dessen 2-Alkylgruppe ein bis acht C-Atome umfaßt, oder 2-Methyl-4,5-trimethylen-4-isothiazolinon enthält.

6. Diagnostischer Testkit nach Anspruch 5,
**dadurch gekennzeichnet,**da**ß**
er 2-Methyl-3(2H)-isothiazolon-hydrochlorid enthält.

7. Konservierungsmittelmischung, enthaltend
a) 0,005 bis 0,1 % Mercaptopyridin-N-oxid-Natriumsalz und
b) 0,005 bis 0,1 % einer der Verbindungen 2-Methyl-3-(2H)-isothiazolonhydrochlorid oder 2-Methyl-4,5-trimethylen-4-isothiazolinon.

8. Konservierungsmittelmischung nach Anspruch 7,
**dadurch gekennzeichnet,** da**ß** sie
a) 0,008 bis 0,012 % Mercaptopyridin-N-oxid-Natriumsalz und
b) 0,015 bis 0,025 % 2-Methyl-3-(2H)- isothiazolon-hydrochlorid enthält.

## Claims

1. Use of mercaptopyridine N-oxide sodium salt together with a compound from the group comprising isothiazolones for preserving test fluids in diagnostic test kits and in particular test fluids in an amylase test.

2. Use according to claim 1,
**characterised in that**
the isothiazolone used is a 2-alkyl-3-(2H)-isothiazolone hydrochloride, whose 2-alkyl group comprises one to eight C atoms, or 2-methyl-4,5-trimethylene-4-isothiazolinone.

3. Use according to claim 2,
**characterised in that**
2-methyl-3-(2H)-isothiazolone hydrochloride is used.

4. Diagnostic test kit,
**characterised in that**
it contains, as preservative for one or more test fluids in the test kit, mercaptopyridine N-oxide sodium salt together with a compound from the group comprising isothiazolones.

5. Diagnostic test kit according to claim 4,
**characterised in that**
it contains, as the compound from the group comprising isothiazolones, a 2-alkyl-3-(2H)-isothiazolone hydrochloride, whose 2-alkyl group comprises one to eight C atoms, or 2-methyl-4,5-trimethylene-4-isothiazolinone.

6. A diagnostic test kit according to claim 5,
**characterised in that**
it contains 2-methyl-3-(2H)-isothiazolone hydrochloride.

7. Preservative mixture, containing
a) 0.005 to 0.1 % mercaptopyridine N-oxide sodium salt and
b) 0.005 to 0.1 % of one of the compounds 2-methyl-3-(2H)-isothiazolone hydrochloride or 2-methyl-4,5-trimethylene-4-isothiazolinone.

8. Preservative mixture according to claim 7,
**characterised in that**
it contains
a) 0.008 to 0.012 % mercaptopyridine N-oxide sodium salt and
b) 0.015 to 0.025 % 2-methyl-3-(2H)-isothiazolone hydrochloride.

## Revendications

1. Utilisation de sel de sodium de mercaptopyridin-N-oxyde conjointement avec un composé du groupe des isothiazolons pour la conservation des liquides de tests de kits ou nécessaires d'essais diagnostiques et en particulier, de liquides de test d'un test d'amylase.

2. Utilisation selon la revendication 1,
**caractérisé en ce qu**' en tant qu'isothiazolon, on met en oeuvre un chlorhydrate de 2-alkyl-3-(2H)-isothiazolon dont le groupe 2-alkyle comprend un jusqu'à huit atomes C, ou 2-méthyl-4,5-triméthylen-4-isothiazolinon.

3. Utilisation selon la revendication 2,
**caractérisé en ce que**,
l'on met en oeuvre le chlorhydrate de 2-méthyl-3-(2H)-isothiazolon.

4. Nécessaire ou kit d'essais diagnostiques,
**caractérisé en ce qu**'il contient en tant qu'agent de conservation un ou plusieurs liquides de tests du nécessaire d'essai de sel de sodium de mercaptopyridin-N-oxyde conjointement avec une composition du groupe des isothiazolons.

5. Nécessaire ou kit d'essais diagnostiques selon la revendication 4,
**caractérisé en ce qu**'il contient en tant que composé du groupe des isothiazolons un chlorhydrate de 2-alkyl-3-(2H)-isothiazolon dont le groupe 2-alkyle contient un jusqu'à huit atomes C, ou 2-méthyl-4,5-triméthylen-4-isothiazolon.

6. Nécessaire ou kit d'essais diagnostiques selon la revendication 5, **caractérisé en ce qu**'il contient un chlorhydrate de 2-méthyl-3-(2H)-isothiazolon.

7. Mélange d'agents de conservation, contenant
a) 0,005 jusqu'à 0,1 % de sel de sodium de mercaptopyridin-N-oxyde. et
b) 0,005 jusqu'à 0,1 % d'un des composés chlorhydrate de 2-méthyl-3-(2H)-isothiazolon ou 2-méthyl-4,5-triméthylen-4-isothiazolon.

8. Mélange d'agents de conservation selon la revendication 7, **caractérisé en ce qu**'il contient
a) 0,008 jusqu'à 0,012% de sel de sodium de mercaptopyridin-N-oxyde et
b) 0,015 jusqu'à 0,025% de chlorhydrate de 2-méthyl-3-(2H)-isothiazolon.
